(19)

Europäisches Patentamt
European Patent Office
Office européen des brevets

(11) **EP 1 832 992 A1**

(12) **EUROPEAN PATENT APPLICATION**
published in accordance with Art. 158(3) EPC

(43) Date of publication:
**12.09.2007 Bulletin 2007/37**

(51) Int Cl.:
***G06F 19/00*** *(2006.01)*

(21) Application number: **04787758.4**

(22) Date of filing: **08.09.2004**

(86) International application number:
**PCT/JP2004/013075**

(87) International publication number:
**WO 2006/027835 (16.03.2006 Gazette 2006/11)**

(84) Designated Contracting States:
**AT BE BG CH CY CZ DE DK EE ES FI FR GB GR HU IE IT LI LU MC NL PL PT RO SE SI SK TR**

(71) Applicant: **GENESYS TECHNOLOGIES, INC.**
**Chuo-ku, Tokyo 103-0023 (JP)**

(72) Inventor: **TANAKA, Junji**
**2470071 (JP)**

(74) Representative: **Möbus, Steffen**
**Kayser & Möbus**
**Patentanwälte**
**Leienfelsstrasse 6**
**81243 München (DE)**

(54) **GENOME ANALYSIS METHOD**

(57)    This invention makes it possible to perform analysis for estimating the characteristics of a population using sample data. By obtaining sample data, embedding genetic (statistical) knowledge in a first and second state variable that have duality, and having the first and second state variables converge to the original value, the characteristics of the population of the sample data are estimated, and the estimated results of the characteristics of the population are output. By doing so, it is possible to perform analysis for estimating characteristics of a population using sample data.

**EP 1 832 992 A1**

**Description**

BACKGROUND OF THE INVENTION

Field of the Invention:

This invention relates to a genome analysis method that performs analysis for estimating the characteristics of a population using sample data.

Description of the Related Art:

All the living organisms existing on earth are made up of cells, and in each individual cell, there are genomes that record gene information. Cells are divided into prokaryotic cells and eukaryotic cells according to differences in their cell structure. Genomes of prokaryotic cells such as bacteria or cyanobacteria exist not in a state of partition by membrane; however, genomes of eukaryotic cells such as in animals and plants exist in a nucleus surrounded by a nuclear membrane.

In other words, a genome indicates a group of chromosomes that are essential for carrying out living activities. Also, the term genome is a compound word that is made from the words gene and chromosome.

Here, the basis of life is the cell, and that cell is surrounded by a cell membrane, and the nucleus is surrounded by a nuclear membrane, such that the independence of each unit is maintained. Human cells comprise specialized cells that can be categorized into nerve cells, muscle cells, blood cells, immune-system cells, epidermal/epithelial cells, which are cells on the surface of the skin and tissue, sensory cells and the like according to function and shape, and undifferentiated cells, called stem cells, which are the source of these cells. Cells have an important aspect that changes with time. That is, cell division and the making of new cells. Cell division is an important mechanism that makes it possible to transmit and express gene information of genes.

There is a chromosome inside the nucleus. That chromosome contains the gene information, and genes are arranged on that chromosome. The genes can also be said to define the method of making protein in a genome. The basic substance that makes up a chromosome is DNA (deoxyribonucleic acid), and the genetic information is preserved in the order of the four bases A, T, G and C in the DNA. A haploid living organism, such as some species of bacteria or virus, has one genome.

A living organism that is a diploid, for example, a reproductive cell such as a human egg or sperm has one set of genomes comprising 23 types of chromosomes. In a somatic cell, there are two groups of genomes (46 types of chromosomes). The human genome comprises approximately 3 billion DNA base pairs (3,000 mega base pairs, 1 mega base pairs equals 1 million base pairs), and when arranged in one string has a length of approximately 1 meter.

A genome is a collection of all of the gene information existing in a cell, and it contains information for controlling the genes and gene expression. Here, protein and genes could be referred to as the product and design drawing, and in the genome, in addition to the design drawing, there exists a part that manages and controls the production of the product. At the present time, the significance of that existence is unclear, however, there is a considerably large percentage of area that is thought to have an effect on maintaining life functions. By clarifying these, it will become possible to gain a more accurate understanding of the life process.

From this, a 'human genome analysis project' for analyzing all human genome base sequences called human genomes, and a project of 'determining all genome base sequences' are being studied for various kinds of organisms, including humans. Also, by performing three-in-one research of genes and protein, it will become possible to gain a high level of understanding of the life process.

In order for that, at first, it is considered that the network between genes must be understood. In other words, a plurality of proteins form a network, and those protein groups carry out a certain function. Therefore, by studying functions or corresponded information, it is possible that a gene having an unknown function may be discovered.

Here, genome analysis is the overall analysis of genetic information contained in the genome of a living organism, and begins from determining the base sequence (GATC sequence) of the DNA molecule of the genome. However, it is not easy to determine the locations and what kind of genes from just the base sequence data. Therefore, analysis of the gene products such as messenger RNA and protein, which is made by transcription and translation, and comparison such as how similar base sequences are between species, and furthermore, and analysis based on data related to individual genes that were analyzed by experimental biology of Escherichia coli, budding yeast or the like.

Incidentally, in the case of humans, the sequence of 3 billion DNA bases that are included in the total 46 chromosomes, 44 autosomal chromosomes, X chromosome and/or Y chromosome (or in other words DNA molecule) is the human genome. The genome information that we have is inherited from the genome information of the parents of the previous generation. The genome information of the parents is inherited from ancestors of even a previous generation. By tracing the origin of genetic information of even previous generations in this way, it is possible to finally reach the genome of the first living organism in 3.8 billion years ago.

As a method of performing genome analysis, patent document 1 discloses a method of analyzing genome in which after genome sequence information is input, determines whether or not there is a sequence section in which a plurality (for example 10 or more) of identical bases are arranged continuously in the input genome sequence information, and when there is a plurality of identical bases, extracts base sequence information that comprises a specified number of bases that are continuously arranged at the front and back of the sequence section in which the plurality of identical bases are arranged, and outputs the extracted base sequence information.

With this kind of method of analyzing genome, it is possible to find polymorphic markers for quickly and efficiently identifying candidate genes related to diseases with accuracy similar to SNPs without using SNPs (single nucleotide polymorphisms).

However, the method disclosed in patent document 1 is a method of analyzing genome that finds polymorphic markers for identifying candidate genes related to diseases; however, in the analysis, it is necessary to analyze the DNA base sequences for approximately 3 billion base pairs from various viewpoints.

Therefore, since it is estimated that there exists various method of analyzing genomes that are still undiscovered, discovery of those methods is anticipated.

SUMMARY OF THE INVENTION

Taking into consideration the above conditions, the object of the present invention is to provide a method of analyzing genome that is capable of estimating the characteristics of a population from sample data.

[Patent Document 1]

Japanese Patent No. 2003-288346

The method of analyzing genome of this invention is a method of analyzing genome for performing analysis for estimating the characteristics of a population from sample data, and comprises: a process of obtaining the sample data; a process of estimating the characteristics of a population to which the sample data belongs by selecting a first and second state variable having duality according to genetic (statistical) knowledge, and making the first and second state variables converge to the original value; and a process of outputting the results of the estimated characteristics of the population.

Also, the method of analyzing genome further comprises a process of mutually performing transformation by transformation equations as operators that are embedded with genetic (statistical) knowledge in which the first and second state variables are expressed by each other, and estimating the first and second state variables by a third state variable that is embedded in those operators.

Moreover, the first state variable is the level of belonging to the original population of each sample, and the second state variable is the haplotype frequency of the original population.

Furthermore, the third state variable is the diplotype and its frequency for each sample.

Also, the method of analyzing genome comprises: a process of setting the gene polymorphism to be investigated; a process of setting allele information by the wet process of the gene polymorphism of the group to be investigated; a process of setting or estimating the haplotype of an individual using the allele information; a processing of setting two characteristic parameters that are in the dual state of the group; a process of developing transformation operators between the two characteristic parameters from the genetic information; a process of starting from a specified initial value and finding the two characteristic parameters in order using transformation operators; and a process of repeating the transformation until the characteristic parameters converge; and wherein the characteristics of a population are estimated from the sample data by finding the two characteristic parameters.

BRIEF EXPLANATION OF THE DRAWINGS

Fig. 1 is a drawing for explaining the genome-analysis apparatus that is used in the method of analyzing genome of this invention.

Fig. 2 is a drawing for explaining the analysis performed by the genome-analysis apparatus shown in Fig. 1.

Fig. 3 is a flowchart showing the method of analyzing genome of this invention.

Fig. 4 is a drawing showing an example of the haplotype frequency of two original populations.

Fig. 5 is a drawing showing $q_i$ evaluation.

Fig. 6 is a drawing showing the original population mixture ratio of an individual for k = 2.

Fig. 7 is a drawing showing the original population mixture ratio of an individual for k = 3.

Fig. 8 is a drawing showing $P_k$ evaluation for k = 2.

Fig. 9 is a drawing showing $P_k$ evaluation for k = 3.

Fig. 10 is a drawing showing comprehensive data as details of the original populations 1 and 2 for k = 2.

Fig. 11 is a drawing showing comprehensive data as details of the original populations 1 to 3 for k = 3.
Fig. 12 is a drawing showing comprehensive data as details of the original populations 1 to 4 for k = 4.
Fig. 13 is a drawing showing comprehensive data as details of $P_k$ evaluation for k = 2.
Fig. 14 is a drawing showing comprehensive data as details of $P_k$ evaluation for k = 2.
Fig. 15 is a drawing showing comprehensive data as details of $P_k$ evaluation for k = 3.

DESCRIPTION OF THE PREFERRED EMBODIMENTS

The preferred embodiments of the invention will be explained below.

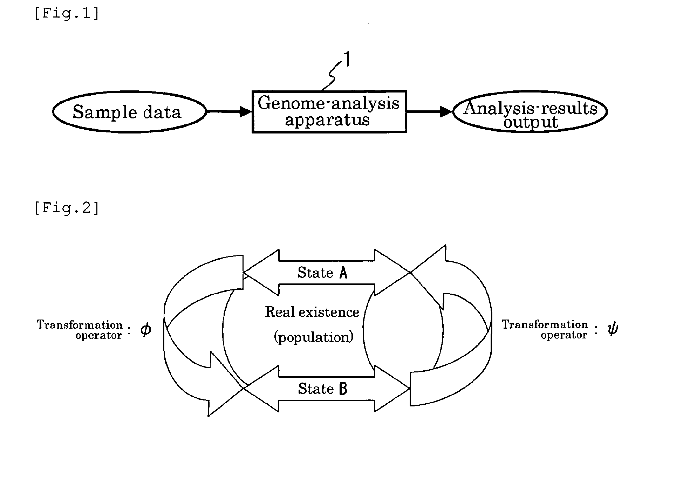

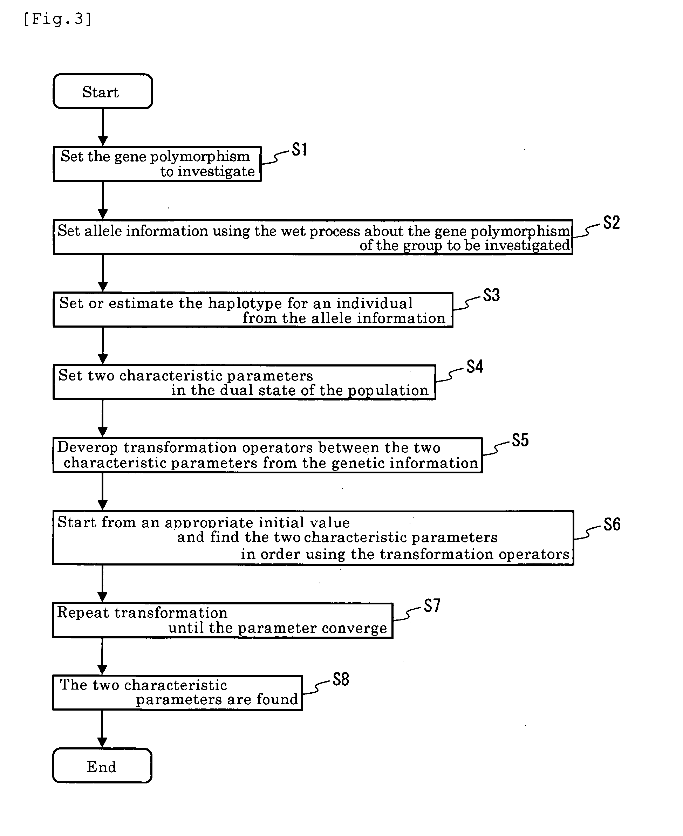

Fig. 1 is a drawing for explaining the genome-analysis apparatus that is used in the method of analyzing genome of this invention; Fig. 2 is a drawing for explaining the analysis performed by the genome-analysis apparatus shown in Fig. 1; and Fig. 3 is a flowchart showing the method of analyzing genome of this invention.
As shown in Fig. 1, the genome-analysis apparatus 1 estimates the characteristics of the population from sample data, and outputs the analysis results. It is possible to use a notebook PC, desktop PC or the like having an analysis program that performs the calculation for the genome analysis (described later) as the genome-analysis apparatus 1.
As shown in Fig. 2, the analysis by the genome-analysis apparatus 1 models real object in which characterization is possible having a state of duality, where the first state is A and second state is B, and by embedding genetic (statistical) knowledge in a transformation operator $\phi$ and transformation operator $\psi$, dual calculation is performed for state A and state B, and by converging to a real (population) value (state), the characteristics of the population are estimated.
Here, state A is the level of belonging to the original population for each sample, and state B is the haplotype frequency of the original population. Also, transformation of state A and state B is mutually performed using transformation equations as operators being expressed by each other, and this will be described in detail later.
Also, in the case where two variables, a first variable and second variable, which express the characteristics of the population to which the sample data belongs, are not completely independent and are also not completely dependent, the genome-analysis apparatus 1 has a function of estimating the two variables from a third variable (incomplete data) that is able to observe these two variables. For example, as shown in Fig. 2, this focuses on being able to consider that state A and state B have a kind of duality.
Therefore, the population to which the sample data belongs is considered to be a system expressible in Hilbert space. Also, for example, the two variables, first and second variable, are taken to be $q_i$ and $p_k$, (where i is the sample number, and k is the original population number). Here, $q_i$ and $p_k$ can be considered as two states that characterize the target system but are not completely independent (entanglement states), or in other words, are a kind of so-called duality. By considering $q_i$ and $p_k$ in this way, they can be considered to be transformation operators that perform mutual transformation of each other such that it is possible to perform Fourier transformation (reverse Fourier transformation) for the particulate aspect and wave aspect of light.
Also, those transformation operators are obtained to be derived from the third variable that is capable of observation, for example, the diplotype of each sample and its frequency $d_i$ (where i is the sample number), and genetic (statistical) knowledge is embedded in those transformation operators. Here, if $q_i$ and $p_k$ actually have duality, then by giving appropriate initial values to $q_i$ and $p_k$, and performing transformation using the operators, they converge to the characteristics of the original population.
As specific examples, it is considered to estimate the original populations from only sample data for the case in which the sample group comprises several original populations.
Here,
the level of belonging to the original population of a sample i is taken to be $q_i$,
the original population is taken to be $k_k$,
the haplotype frequency of the original population k is taken to be $p_k$, and
the diplotype frequency of sample i is taken to be $d_i$.
Also, $q_i$, $p_k$ and $d_i$ can be expressed as follows:

$$[1]$$

$$q_i = \sum_j c_{ik} | K_k > \quad : \quad \text{where,} \sum_j c_{ik} = 1$$

[2]

$$d_i = \Sigma_{ll'} a_{ill'} |h_{il}> |h_{il'}> \quad : \text{where} \ \Sigma_{ll'} a_{ill'} = 1$$

[3]

$$p_k = \Sigma_m b_{km} |h_{km}> \quad : \text{where} \ \Sigma_m b_{km} = 1$$

Here, $|K_k>$ (original population vector), and $|h_{km}>$, $|h_{i1}>$, and $|h_{i1'}>$ (haplotype vectors) can each be considered to be one base vector of the vector space to which the sample group belongs.

Here, p and q transform each other with a projection operator, and can be expressed as shown below.

[4]

$$q = \psi p \quad : \text{where} \ \psi \ \text{is a projection operator.}$$

[5]

$$p = \phi q \quad : \text{where} \ \phi \ \text{is a projection operator}$$

Here the actual operators can be considered as shown below.

[6]

$$\psi_{ik} = \Sigma_{ll'} a_{ill'} {}^* b_{kl} {}^* b_{kl'}$$

[7]

$$\phi k = \Sigma_i c_{ik} \Sigma_{ll'} a_{ill'}$$

In other words, the operators $\phi$ and $\psi$ can be considered as a system that can express the population to which the sample belongs in Hilbert space, and $q_i$ and $p_k$ characterize the target system, and can be considered to express two states that are not completely independent (entanglement states), and can be handled as a kind of so-called duality.

From these considerations, it is possible to consider $q_i$ and $p_k$ as being operators that transform each other, and these operators can be derived from $d_i$, and if it is possible to find $q_i$ and $p_k$ in order, it is possible to converge on the original value (state) of the population.

Also, the operators $\phi$ and $p_k$ are added for items for which $|h_i>$ of each sample, and $|h_k>$ of each group match with the probability $c_k$ for each k (original population), and this can be considered to be the same as standardization. Moreover, the operators $\psi$ and $q_i$ are added for each k according to $b_k$ of matching $|h_i>$ and $|h_k>$ at the ratio $a_i$ of simultaneous probability of $|h_{ij1}>$ and $|h_{ij2}>$, and this can be considered to be the same as standardization. Therefore, by starting from an appropriate initial state, q and p are found using the procedure described above, and converge. Determining whether or not they converge can be performed by determining whether or not p and q converge to a certain value.

Then, the method of analyzing genome by the genome-analysis apparatus 1 will be explained.

At first, as shown in Fig. 3, it is determined the gene polymorphism to be investigated (step S1). Here, firstly, allele information for the gene polymorphism of the group to be investigated is determined by using a wet process (step S2). Also, the haplotype of an individual is determined or estimated by the allele information (step S3).

Next, the two characteristic parameters in the dual state of the population are determined (step S4). Here, these two characteristic parameters are the level of belonging to the original population of the sample and the haplotype frequency of each original population. Also, transformation operators are developed between the two characteristic parameters by using the genetic information (step S5). The genetic information here is the diplotype of an individual and its frequency.

Then, starting from adequate initial values, the two characteristic parameters are determined in turn by using the transformation operators (step S6). Then, transformation is repeated until the parameters converge (step S7). After that, the two characteristic parameters are determined (step S8).

(Embodiment)

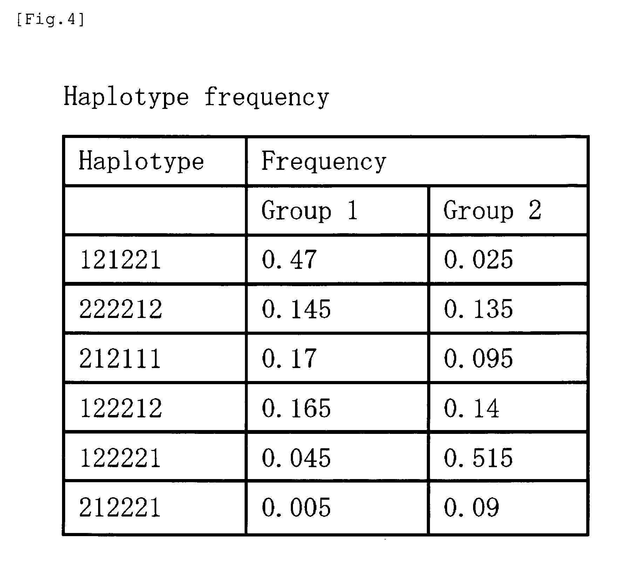

Next, an embodiment will be explained.
The figures from Fig. 4 to Fig. 15 show an example of the analysis results of the method of analyzing genome by using transformation operators that have duality and that use genotype data and haplotype data for a plurality of locus in order to deduce the original population and assign each sample to the original population.

In gene analysis, case-control-correlation analysis is a powerful method for mapping the genotype data on the phenotype data (for example, correlation mapping for finding disease genes). However, when estimating the original population, there is a possibility that an error will occur in mapping the genotype data from the structured group and that it will result in a positive result by using case-control-correlation analysis.

Therefore, before performing case-control-correlation analysis, it is preferred that the potential group structure be detected. To detect the potential group structure, there is the MCMC method based on Bayesian statistics, or a method that uses a position allele, such as a class model that is based on the concept of distance between samples, to identify the structured group, however, in this embodiment, a new modeling method will be employed that uses a dual transformation operator algorithm.

In this case, the haplotypes are considered to be more informative gene information than allele, so haplotypes are used instead of allele. Also, vectors in Hilbert space and their operators are used in the case-control-correlation analysis of the gene analysis of the group structure. In other words, this is because it was presumed that there is hidden real existence belonging to the sampled individual.

Here, the vectors in Hilbert space express the genetic state. Also, the operators can be transformed from one vector expression to another vector expression.

Therefore, when the two variables that express the characteristics of the population to which the sample data belongs are not completely independent, and are also not completely dependent, a method of estimating the two variables from a third variable (incomplete data) that is capable of observing the two variables is employed.

In this embodiment, as described above, $p_k$, the haplotype frequency of the original population, and $q_i$, the level of belonging to the original population of the sample, are used as two characterizing operators that exist in a dual state. By doing this, it is considered that the hidden real object belonging to the sampled individual is estimated. Moreover, in this embodiment, as described above, the diplotype of the individual and its frequency $d_i$ are used as observed data.

Here, as described above, $q_i$ and $p_k$ are two states (entanglement states) that characterize the target system and that are not completely independent, and are considered to be a kind of so-called duality. By making this consideration, as described above, $q_i$ and $p_k$ can be considered to be transformation operators that perform mutual transformation of each other such that it is possible to perform Fourier transformation (reverse Fourier transformation) for the particulate aspect and wave aspect of light.

[8]

$$q_i = \phi(p_k) \quad \dots \quad (1)$$

[9]

$$p_k = \psi(q_i) \quad \dots \quad (2)$$

Therefore, Equation (1) and Equation (2) are assumed for $q_i$ and $p_k$, and these operators are estimated from genetic statistical knowledge.

Also, by taking the diplotype of the individual and its frequency to be $d_i$, it can be expressed as in the following equations (3) to (5) in Hilbert space expression.

[10]

$$qi = \Sigma_j c_{ik} \mid K_k > \qquad where \ \Sigma_k c_{ik} = 1 \ ......(3)$$

[11]

$$h_i = \Sigma_{11'} a_{i11'} \mid h_{i1} > \mid h_{i1'} > \quad where \Sigma_{11'} a_{i11'} = 1 \ ...... (4)$$

[12]

$$p_k = \Sigma_m b_{km} \mid h_{km} > \qquad where \Sigma_m b_{km} = 1 \ ....... (5)$$

Further, $|K_k>$ (original population vector), and $|h_{km}>, | h_{i1}>$, and $|h_{i1'}>$ (haplotype vectors) can each be considered to be one base vector of the vector space to which the sample group belongs.

Also, the following equations (6) and (7) are used as actual duality transformation operators.

[13]

$$\psi_{ik} = \Sigma_{11'} a_{i11'} {}^* b_{k1} {}^* b_{k1'} \ ....... (6)$$

[14]

$$\phi_k = \Sigma_i c_{ik} \Sigma_{11'} a_{i11'} \ ...... (7)$$

Then, from these equations, firstly, in step 1), an appropriate initial value is set for $q_i$ from $d_i$. However, the initial value is except for 1/k. Also, k is the number of the original population. Then, in step 2), $p_k$ is determined from equation (7) . Then, in step 3), $q_i$ is determined from equation (6). Here, calculation is repeated until $p_k$ and $q_i$ converge.

Next, the haplotype frequency data for each original population of a structured population will be explained.

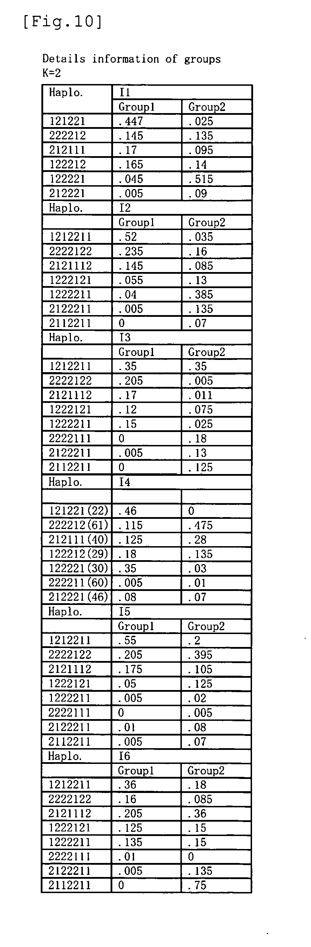

Fig. 4 shows an example of the haplotype frequency for, as for example, two groups of the group (original population). In this example, the haplotype is expressed from six loci. Also, it can be seen that each locus has two allelic genes (SNP). Here, "1" indicates a large number of allelic genes, and "2" indicates a small number of allelic genes. The detailed group (original population) information and haplotype frequency evaluated here can be checked from the comprehensive data shown in Fig. 10.

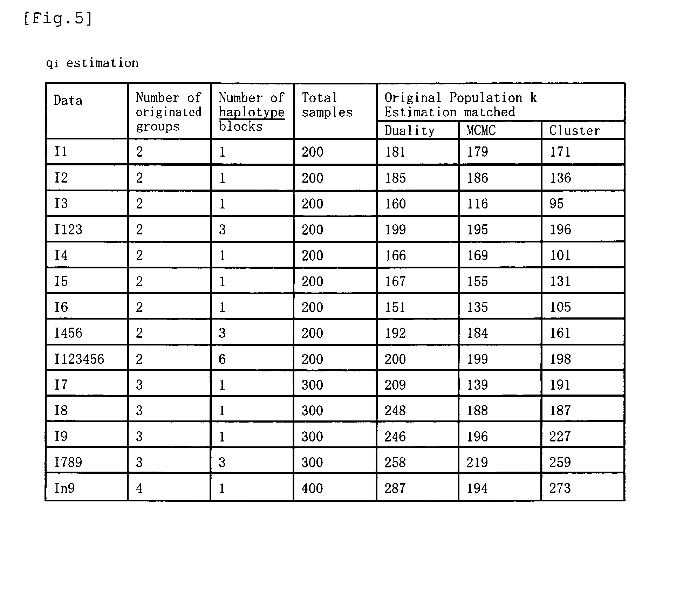

Fig. 5 shows the $q_i$ evaluation, and the details of this can be checked from the comprehensive data shown in Fig. 10. Here, it is shown that the number of original populations comprised for the sampled population and a comparison of the evaluation between the method of this invention and other method. Here, it is difficult to distinguish these differences if the haplotype frequencies of the original population are more similar; however, as the number of haplotype blocks increases, the better results can be obtained.

For example, I123 is a data for a combination of the three haplotype blocks I1, I2, and I3. Moreover, I123456 is data for a combination of I1, I2, I3, I4, I5, and I6. The result of these plurality of haplotype blocks give a better agreement than for a single block unit.

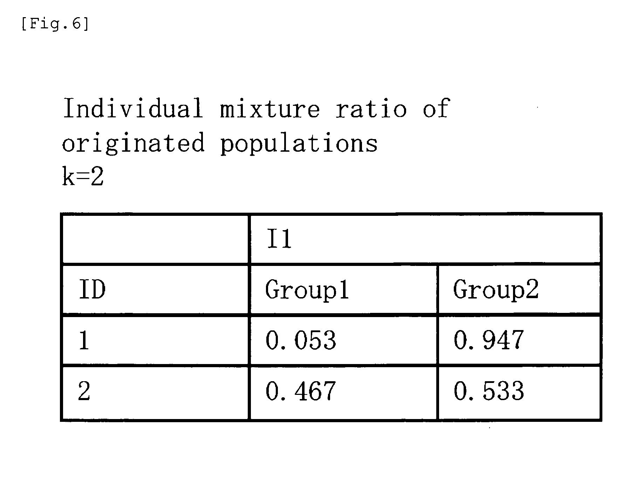

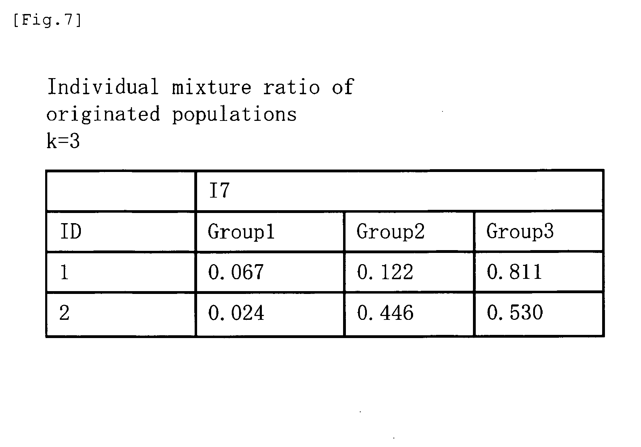

Fig. 6 shows original population mixture ratios for a sample when k (number of original populations) = 2, and Fig. 7 shows original population mixture ratios for a sample when k (number of original populations) = 3. In other words, when the original population mixture ratio for a sample is "1", the sample belongs to one group, however, when the mixture ratio is between 0 and 1, the sample belongs to a plurality of original populations at that mixture ratio.

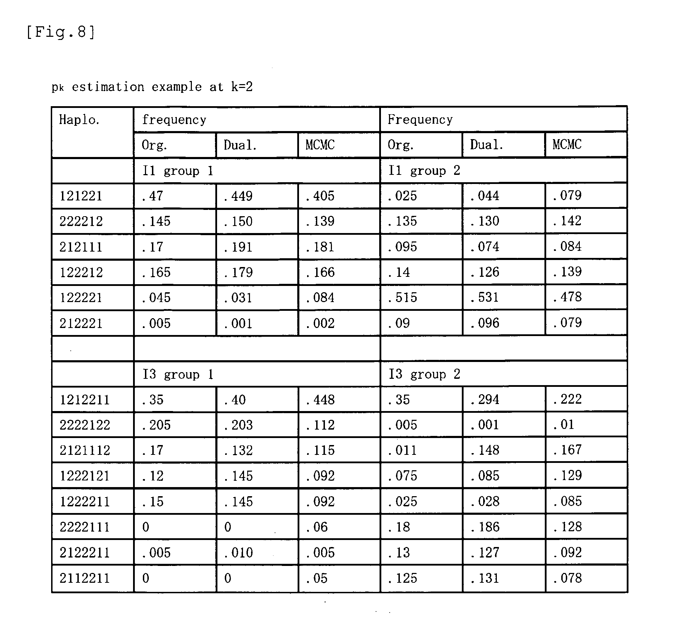

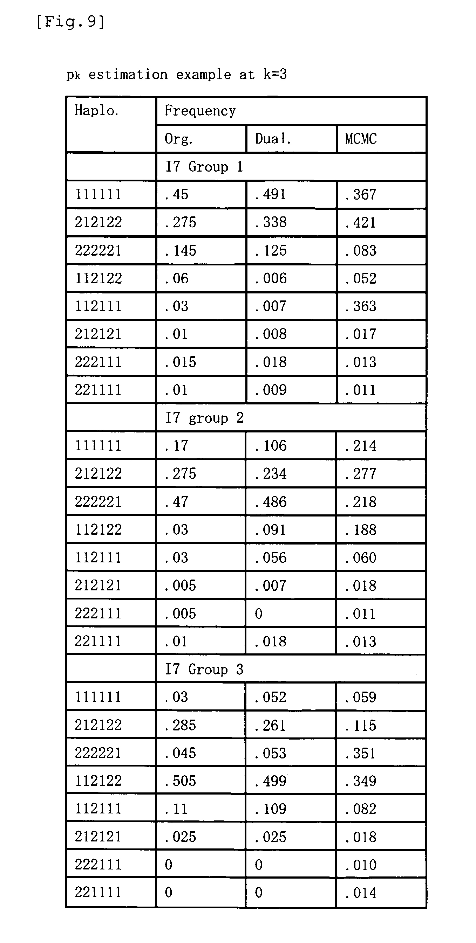

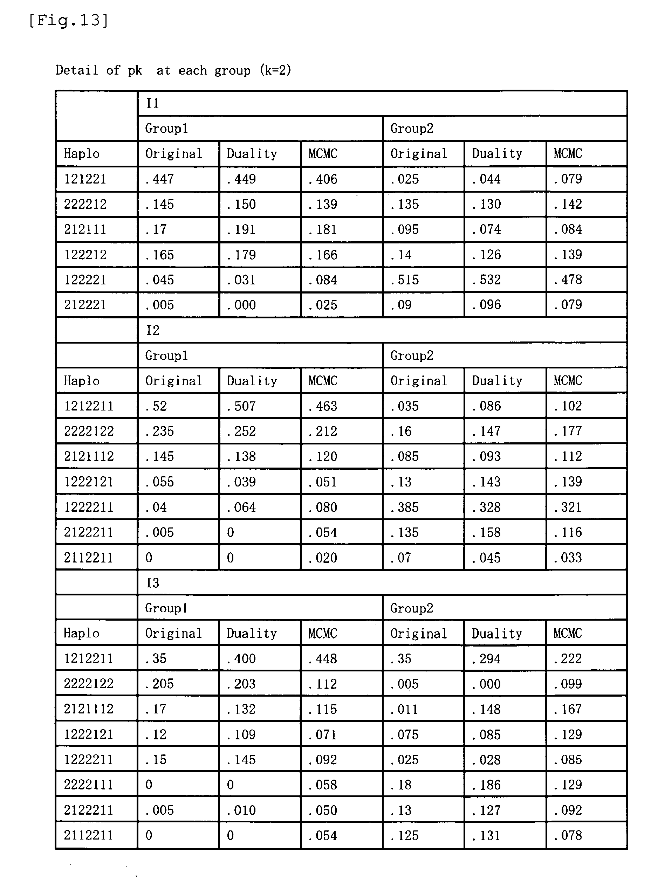

Fig. 8 shows the $p_k$ evaluation when k = 2, and Fig. 9 shows the $p_k$ evaluation when k = 3. It can be seen that with evaluation using duality transformation, it is shown that the results are identical or better than the results by using the MCMC method. The $p_k$ evaluation can be checked from the comprehensive data shown in Fig. 13 to Fig. 15.

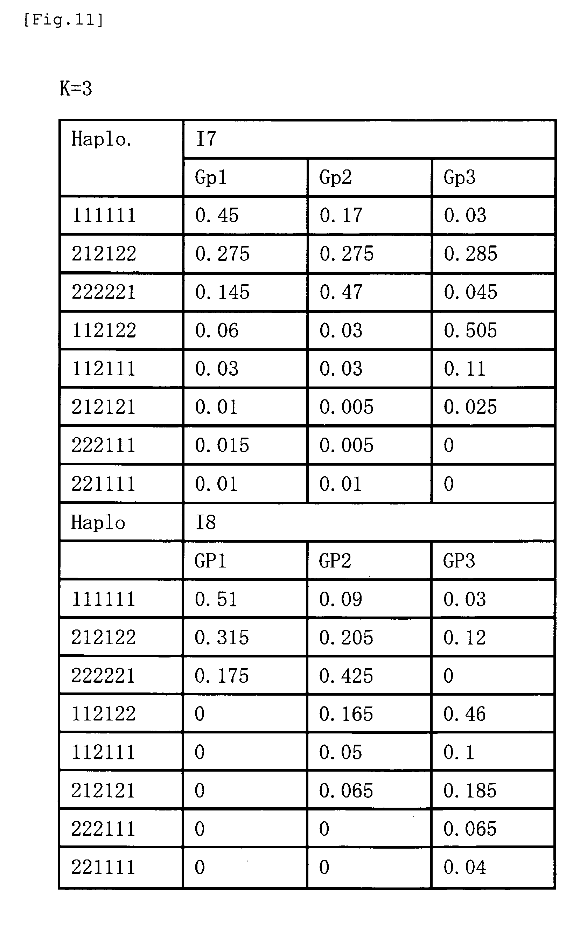

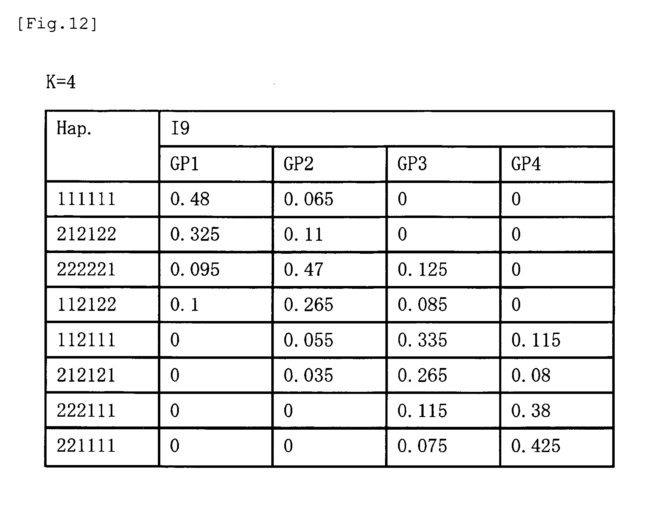

Here, Fig. 10 is a drawing showing comprehensive data, which gives details of the original population groups 1 and 2 when k = 2, Fig. 11 is a drawing showing comprehensive data, which gives details of the original population groups 1 to 3 when k = 3, and Fig. 12 is a drawing showing comprehensive data, which gives details of the original population groups 1 to 4 when k = 4.

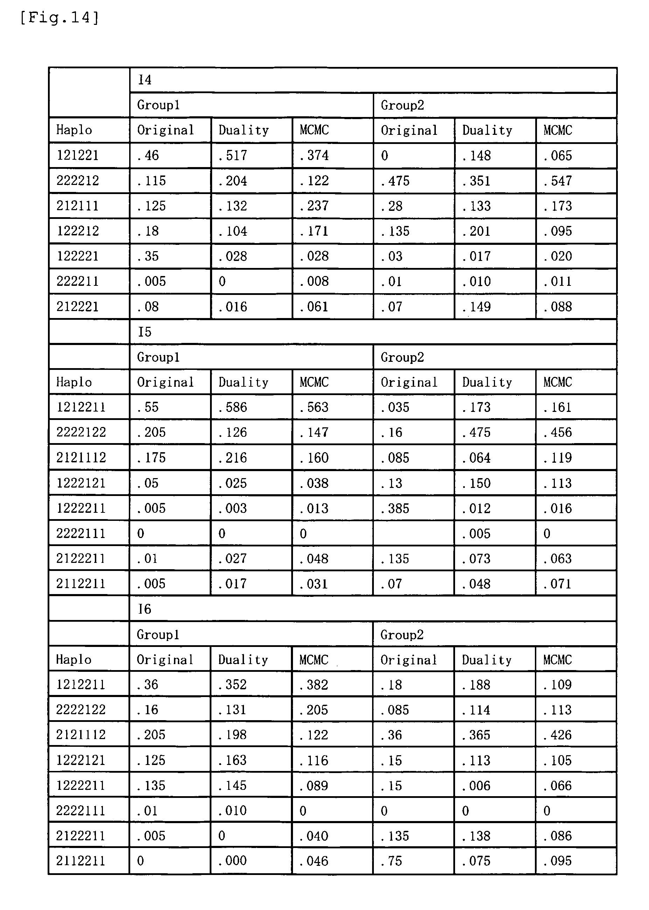

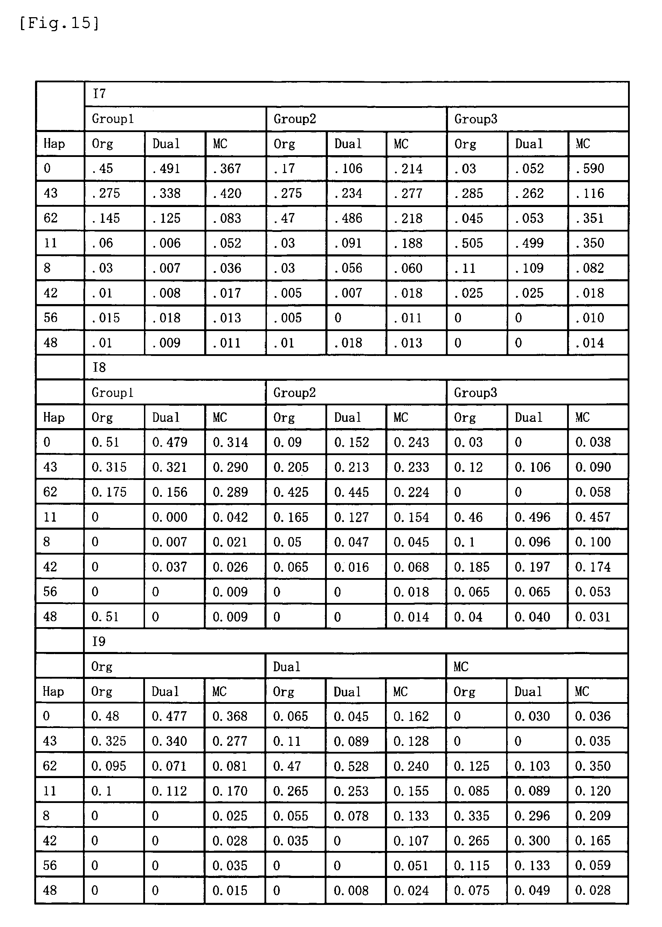

Also, Fig. 13 and Fig. 14 show comprehensive data, which gives details of $p_k$ evaluation when k = 2, and Fig. 15 shows comprehensive data, which gives details of $p_k$ evaluation when k = 3.

In this way, in this embodiment, by obtaining sample data, embedding genetic (statistical) knowledge in a first and second state variable that have duality, and having the first and second state variables converge to what the original values should be, it is possible to estimate the characteristics of the population of the sample data, and output the estimated results of the characteristics of the population, so it is possible to perform analysis for estimating characteristics of a population from sample data.

[Industrial Applicability]

As described above, with this invention it is possible to perform analysis for estimating characteristics of a population from sample data.

**Claims**

1. A method of analyzing genome wherein performs analysis for estimating the characteristics of a population from sample data, the method comprising the steps of:

   obtaining said sample data;
   estimating the characteristics of a population to which said sample data belongs to by selecting a first and second state variable having duality according to genetic (statistical) knowledge, and making said first and second state variables converge to the value what the original value should be; and
   outputting the results of said estimated characteristics of said population.

2. The method of analyzing genome of claim 1, wherein the method further comprising the steps of:

   mutually performing transformation by transformation equations as operators that are embedded with genetic (statistical) knowledge in which said first and second state variables are expressed by each other, and estimating said first and second state variables by a third state variable that is embedded in those operators.

3. The method of analyzing genome of claim 1 or claim 2, wherein said first state variable is the level of belonging to the original population of each sample, and said second state variable is the haplotype frequency of the original population.

4. The method of analyzing genome of any one of the claims 1 to 3, wherein said third state variable is the diplotype and its frequency for each sample.

5. The method of analyzing genome of any one of the claims 1 to 4, wherein the method further comprising the steps of:

   determining the gene polymorphism to be investigated;
   determining allele information by a wet process of the gene polymorphism of the group to be investigated;
   determining or estimating the haplotype of an individual using said allele information;
   determining two characteristic parameters that are in the dual state of the group;
   developing transformation operators between said two characteristic parameters from the genetic information;
   starting from a specified initial value and finding said two characteristic parameters in order by using the transformation operators; and
   repeating the transformation until said characteristic parameters converge; and wherein

the characteristics of a population are estimated from said sample data by determining said two characteristic parameters.

[Fig.1]

[Fig.2]

[Fig.3]

```
                    ┌──────────────┐
                    │    Start     │
                    └──────┬───────┘
                           │
                           ▼
          ┌──────────────────────────┐
          │ Set the gene polymorphism│ ⟋ S1
          │            to investigate│
          └──────────┬───────────────┘
                     │
                     ▼
┌────────────────────────────────────────────────────────┐
│ Set allele information using the wet process about the  │ ⟋ S2
│ gene polymorphism of the group to be investigated       │
└──────────┬─────────────────────────────────────────────┘
           │
           ▼
┌────────────────────────────────────────────┐
│ Set or estimate the haplotype for an       │ ⟋ S3
│ individual from the allele information      │
└──────────┬─────────────────────────────────┘
           │
           ▼
┌────────────────────────────────────────────┐
│ Set two characteristic parameters          │ ⟋ S4
│ in the dual state of the population         │
└──────────┬─────────────────────────────────┘
           │
           ▼
┌────────────────────────────────────────────┐
│ Deverop transformation operators between   │ ⟋ S5
│ the two characteristic parameters from the │
│ genetic information                         │
└──────────┬─────────────────────────────────┘
           │
           ▼
┌──────────────────────────────────────────────────┐
│ Start from an appropriate initial value          │ ⟋ S6
│ and find the two characteristic parameters        │
│ in order using the transformation operators       │
└──────────┬───────────────────────────────────────┘
           │
           ▼
┌────────────────────────────────────────────┐
│ Repeat transformation                      │ ⟋ S7
│ until the parameter converge                │
└──────────┬─────────────────────────────────┘
           │
           ▼
┌────────────────────────────────────────────┐
│ The two characteristic                     │ ⟋ S8
│ parameters are found                        │
└──────────┬─────────────────────────────────┘
           │
           ▼
    ┌──────────────┐
    │     End      │
    └──────────────┘
```

[Fig.4]

Haplotype frequency

| Haplotype | Frequency | |
|---|---|---|
| | Group 1 | Group 2 |
| 121221 | 0. 47 | 0. 025 |
| 222212 | 0. 145 | 0. 135 |
| 212111 | 0. 17 | 0. 095 |
| 122212 | 0. 165 | 0. 14 |
| 122221 | 0. 045 | 0. 515 |
| 212221 | 0. 005 | 0. 09 |

[Fig.5]

qi estimation

| Data | Number of originated groups | Number of haplotype blocks | Total samples | Original Population k Estimation matched | | |
|------|------|------|------|------|------|------|
| | | | | Duality | MCMC | Cluster |
| I1 | 2 | 1 | 200 | 181 | 179 | 171 |
| I2 | 2 | 1 | 200 | 185 | 186 | 136 |
| I3 | 2 | 1 | 200 | 160 | 116 | 95 |
| I123 | 2 | 3 | 200 | 199 | 195 | 196 |
| I4 | 2 | 1 | 200 | 166 | 169 | 101 |
| I5 | 2 | 1 | 200 | 167 | 155 | 131 |
| I6 | 2 | 1 | 200 | 151 | 135 | 105 |
| I456 | 2 | 3 | 200 | 192 | 184 | 161 |
| I123456 | 2 | 6 | 200 | 200 | 199 | 198 |
| I7 | 3 | 1 | 300 | 209 | 139 | 191 |
| I8 | 3 | 1 | 300 | 248 | 188 | 187 |
| I9 | 3 | 1 | 300 | 246 | 196 | 227 |
| I789 | 3 | 3 | 300 | 258 | 219 | 259 |
| In9 | 4 | 1 | 400 | 287 | 194 | 273 |

[Fig.6]

Individual mixture ratio of
originated populations
k=2

|  | I1 | |
| --- | --- | --- |
| ID | Group1 | Group2 |
| 1 | 0. 053 | 0. 947 |
| 2 | 0. 467 | 0. 533 |

[Fig.7]

Individual mixture ratio of
originated populations
k=3

|  | I7 | | |
|---|---|---|---|
| ID | Group1 | Group2 | Group3 |
| 1 | 0.067 | 0.122 | 0.811 |
| 2 | 0.024 | 0.446 | 0.530 |

[Fig.8]

p$_k$ estimation example at k=2

| Haplo. | frequency | | | Frequency | | |
|---|---|---|---|---|---|---|
| | Org. | Dual. | MCMC | Org. | Dual. | MCMC |
| | I1 group 1 | | | I1 group 2 | | |
| 121221 | .47 | .449 | .405 | .025 | .044 | .079 |
| 222212 | .145 | .150 | .139 | .135 | .130 | .142 |
| 212111 | .17 | .191 | .181 | .095 | .074 | .084 |
| 122212 | .165 | .179 | .166 | .14 | .126 | .139 |
| 122221 | .045 | .031 | .084 | .515 | .531 | .478 |
| 212221 | .005 | .001 | .002 | .09 | .096 | .079 |
| | | | | | | |
| | I3 group 1 | | | I3 group 2 | | |
| 1212211 | .35 | .40 | .448 | .35 | .294 | .222 |
| 2222122 | .205 | .203 | .112 | .005 | .001 | .01 |
| 2121112 | .17 | .132 | .115 | .011 | .148 | .167 |
| 1222121 | .12 | .145 | .092 | .075 | .085 | .129 |
| 1222211 | .15 | .145 | .092 | .025 | .028 | .085 |
| 2222111 | 0 | 0 | .06 | .18 | .186 | .128 |
| 2122211 | .005 | .010 | .005 | .13 | .127 | .092 |
| 2112211 | 0 | 0 | .05 | .125 | .131 | .078 |

[Fig.9]

pk estimation example at k=3

| Haplo. | Frequency | | |
|---|---|---|---|
| | Org. | Dual. | MCMC |
| | I7 Group 1 | | |
| 111111 | .45 | .491 | .367 |
| 212122 | .275 | .338 | .421 |
| 222221 | .145 | .125 | .083 |
| 112122 | .06 | .006 | .052 |
| 112111 | .03 | .007 | .363 |
| 212121 | .01 | .008 | .017 |
| 222111 | .015 | .018 | .013 |
| 221111 | .01 | .009 | .011 |
| | I7 group 2 | | |
| 111111 | .17 | .106 | .214 |
| 212122 | .275 | .234 | .277 |
| 222221 | .47 | .486 | .218 |
| 112122 | .03 | .091 | .188 |
| 112111 | .03 | .056 | .060 |
| 212121 | .005 | .007 | .018 |
| 222111 | .005 | 0 | .011 |
| 221111 | .01 | .018 | .013 |
| | I7 Group 3 | | |
| 111111 | .03 | .052 | .059 |
| 212122 | .285 | .261 | .115 |
| 222221 | .045 | .053 | .351 |
| 112122 | .505 | .499 | .349 |
| 112111 | .11 | .109 | .082 |
| 212121 | .025 | .025 | .018 |
| 222111 | 0 | 0 | .010 |
| 221111 | 0 | 0 | .014 |

[Fig.10]

Details information of groups
K=2

| Haplo. | I1 | |
|---|---|---|
| | Group1 | Group2 |
| 121221 | .447 | .025 |
| 222212 | .145 | .135 |
| 212111 | .17 | .095 |
| 122212 | .165 | .14 |
| 122221 | .045 | .515 |
| 212221 | .005 | .09 |
| Haplo. | I2 | |
| | Group1 | Group2 |
| 1212211 | .52 | .035 |
| 2222122 | .235 | .16 |
| 2121112 | .145 | .085 |
| 1222121 | .055 | .13 |
| 1222211 | .04 | .385 |
| 2122211 | .005 | .135 |
| 2112211 | 0 | .07 |
| Haplo. | I3 | |
| | Group1 | Group2 |
| 1212211 | .35 | .35 |
| 2222122 | .205 | .005 |
| 2121112 | .17 | .011 |
| 1222121 | .12 | .075 |
| 1222211 | .15 | .025 |
| 2222111 | 0 | .18 |
| 2122211 | .005 | .13 |
| 2112211 | 0 | .125 |
| Haplo. | I4 | |
| | | |
| 121221(22) | .46 | 0 |
| 222212(61) | .115 | .475 |
| 212111(40) | .125 | .28 |
| 122212(29) | .18 | .135 |
| 122221(30) | .35 | .03 |
| 222211(60) | .005 | .01 |
| 212221(46) | .08 | .07 |
| Haplo. | I5 | |
| | Group1 | Group2 |
| 1212211 | .55 | .2 |
| 2222122 | .205 | .395 |
| 2121112 | .175 | .105 |
| 1222121 | .05 | .125 |
| 1222211 | .005 | .02 |
| 2222111 | 0 | .005 |
| 2122211 | .01 | .08 |
| 2112211 | .005 | .07 |
| Haplo. | I6 | |
| | Group1 | Group2 |
| 1212211 | .36 | .18 |
| 2222122 | .16 | .085 |
| 2121112 | .205 | .36 |
| 1222121 | .125 | .15 |
| 1222211 | .135 | .15 |
| 2222111 | .01 | 0 |
| 2122211 | .005 | .135 |
| 2112211 | 0 | .75 |

[Fig.11]

K=3

| Haplo. | I7 | | |
|---|---|---|---|
| | Gp1 | Gp2 | Gp3 |
| 111111 | 0. 45 | 0. 17 | 0. 03 |
| 212122 | 0. 275 | 0. 275 | 0. 285 |
| 222221 | 0. 145 | 0. 47 | 0. 045 |
| 112122 | 0. 06 | 0. 03 | 0. 505 |
| 112111 | 0. 03 | 0. 03 | 0. 11 |
| 212121 | 0. 01 | 0. 005 | 0. 025 |
| 222111 | 0. 015 | 0. 005 | 0 |
| 221111 | 0. 01 | 0. 01 | 0 |
| Haplo | I8 | | |
| | GP1 | GP2 | GP3 |
| 111111 | 0. 51 | 0. 09 | 0. 03 |
| 212122 | 0. 315 | 0. 205 | 0. 12 |
| 222221 | 0. 175 | 0. 425 | 0 |
| 112122 | 0 | 0. 165 | 0. 46 |
| 112111 | 0 | 0. 05 | 0. 1 |
| 212121 | 0 | 0. 065 | 0. 185 |
| 222111 | 0 | 0 | 0. 065 |
| 221111 | 0 | 0 | 0. 04 |

[Fig.12]

K=4

| Hap. | I9 | | | |
|---|---|---|---|---|
| | GP1 | GP2 | GP3 | GP4 |
| 111111 | 0. 48 | 0. 065 | 0 | 0 |
| 212122 | 0. 325 | 0. 11 | 0 | 0 |
| 222221 | 0. 095 | 0. 47 | 0. 125 | 0 |
| 112122 | 0. 1 | 0. 265 | 0. 085 | 0 |
| 112111 | 0 | 0. 055 | 0. 335 | 0. 115 |
| 212121 | 0 | 0. 035 | 0. 265 | 0. 08 |
| 222111 | 0 | 0 | 0. 115 | 0. 38 |
| 221111 | 0 | 0 | 0. 075 | 0. 425 |

[Fig.13]

Detail of pk  at each group (k=2)

| | I1 | | | | | |
|---|---|---|---|---|---|---|
| | Group1 | | | Group2 | | |
| Haplo | Original | Duality | MCMC | Original | Duality | MCMC |
| 121221 | .447 | .449 | .406 | .025 | .044 | .079 |
| 222212 | .145 | .150 | .139 | .135 | .130 | .142 |
| 212111 | .17 | .191 | .181 | .095 | .074 | .084 |
| 122212 | .165 | .179 | .166 | .14 | .126 | .139 |
| 122221 | .045 | .031 | .084 | .515 | .532 | .478 |
| 212221 | .005 | .000 | .025 | .09 | .096 | .079 |
| | I2 | | | | | |
| | Group1 | | | Group2 | | |
| Haplo | Original | Duality | MCMC | Original | Duality | MCMC |
| 1212211 | .52 | .507 | .463 | .035 | .086 | .102 |
| 2222122 | .235 | .252 | .212 | .16 | .147 | .177 |
| 2121112 | .145 | .138 | .120 | .085 | .093 | .112 |
| 1222121 | .055 | .039 | .051 | .13 | .143 | .139 |
| 1222211 | .04 | .064 | .080 | .385 | .328 | .321 |
| 2122211 | .005 | 0 | .054 | .135 | .158 | .116 |
| 2112211 | 0 | 0 | .020 | .07 | .045 | .033 |
| | I3 | | | | | |
| | Group1 | | | Group2 | | |
| Haplo | Original | Duality | MCMC | Original | Duality | MCMC |
| 1212211 | .35 | .400 | .448 | .35 | .294 | .222 |
| 2222122 | .205 | .203 | .112 | .005 | .000 | .099 |
| 2121112 | .17 | .132 | .115 | .011 | .148 | .167 |
| 1222121 | .12 | .109 | .071 | .075 | .085 | .129 |
| 1222211 | .15 | .145 | .092 | .025 | .028 | .085 |
| 2222111 | 0 | 0 | .058 | .18 | .186 | .129 |
| 2122211 | .005 | .010 | .050 | .13 | .127 | .092 |
| 2112211 | 0 | 0 | .054 | .125 | .131 | .078 |

[Fig.14]

| | I4 | | | | | |
|---|---|---|---|---|---|---|
| | Group1 | | | Group2 | | |
| Haplo | Original | Duality | MCMC | Original | Duality | MCMC |
| 121221 | .46 | .517 | .374 | 0 | .148 | .065 |
| 222212 | .115 | .204 | .122 | .475 | .351 | .547 |
| 212111 | .125 | .132 | .237 | .28 | .133 | .173 |
| 122212 | .18 | .104 | .171 | .135 | .201 | .095 |
| 122221 | .35 | .028 | .028 | .03 | .017 | .020 |
| 222211 | .005 | 0 | .008 | .01 | .010 | .011 |
| 212221 | .08 | .016 | .061 | .07 | .149 | .088 |
| | I5 | | | | | |
| | Group1 | | | Group2 | | |
| Haplo | Original | Duality | MCMC | Original | Duality | MCMC |
| 1212211 | .55 | .586 | .563 | .035 | .173 | .161 |
| 2222122 | .205 | .126 | .147 | .16 | .475 | .456 |
| 2121112 | .175 | .216 | .160 | .085 | .064 | .119 |
| 1222121 | .05 | .025 | .038 | .13 | .150 | .113 |
| 1222211 | .005 | .003 | .013 | .385 | .012 | .016 |
| 2222111 | 0 | 0 | 0 | | .005 | 0 |
| 2122211 | .01 | .027 | .048 | .135 | .073 | .063 |
| 2112211 | .005 | .017 | .031 | .07 | .048 | .071 |
| | I6 | | | | | |
| | Group1 | | | Group2 | | |
| Haplo | Original | Duality | MCMC | Original | Duality | MCMC |
| 1212211 | .36 | .352 | .382 | .18 | .188 | .109 |
| 2222122 | .16 | .131 | .205 | .085 | .114 | .113 |
| 2121112 | .205 | .198 | .122 | .36 | .365 | .426 |
| 1222121 | .125 | .163 | .116 | .15 | .113 | .105 |
| 1222211 | .135 | .145 | .089 | .15 | .006 | .066 |
| 2222111 | .01 | .010 | 0 | 0 | 0 | 0 |
| 2122211 | .005 | 0 | .040 | .135 | .138 | .086 |
| 2112211 | 0 | .000 | .046 | .75 | .075 | .095 |

[Fig.15]

| | I7 | | | | | | | | |
|---|---|---|---|---|---|---|---|---|---|
| | Group1 | | | Group2 | | | Group3 | | |
| Hap | Org | Dual | MC | Org | Dual | MC | Org | Dual | MC |
| 0 | .45 | .491 | .367 | .17 | .106 | .214 | .03 | .052 | .590 |
| 43 | .275 | .338 | .420 | .275 | .234 | .277 | .285 | .262 | .116 |
| 62 | .145 | .125 | .083 | .47 | .486 | .218 | .045 | .053 | .351 |
| 11 | .06 | .006 | .052 | .03 | .091 | .188 | .505 | .499 | .350 |
| 8 | .03 | .007 | .036 | .03 | .056 | .060 | .11 | .109 | .082 |
| 42 | .01 | .008 | .017 | .005 | .007 | .018 | .025 | .025 | .018 |
| 56 | .015 | .018 | .013 | .005 | 0 | .011 | 0 | 0 | .010 |
| 48 | .01 | .009 | .011 | .01 | .018 | .013 | 0 | 0 | .014 |
| | I8 | | | | | | | | |
| | Group1 | | | Group2 | | | Group3 | | |
| Hap | Org | Dual | MC | Org | Dual | MC | Org | Dual | MC |
| 0 | 0.51 | 0.479 | 0.314 | 0.09 | 0.152 | 0.243 | 0.03 | 0 | 0.038 |
| 43 | 0.315 | 0.321 | 0.290 | 0.205 | 0.213 | 0.233 | 0.12 | 0.106 | 0.090 |
| 62 | 0.175 | 0.156 | 0.289 | 0.425 | 0.445 | 0.224 | 0 | 0 | 0.058 |
| 11 | 0 | 0.000 | 0.042 | 0.165 | 0.127 | 0.154 | 0.46 | 0.496 | 0.457 |
| 8 | 0 | 0.007 | 0.021 | 0.05 | 0.047 | 0.045 | 0.1 | 0.096 | 0.100 |
| 42 | 0 | 0.037 | 0.026 | 0.065 | 0.016 | 0.068 | 0.185 | 0.197 | 0.174 |
| 56 | 0 | 0 | 0.009 | 0 | 0 | 0.018 | 0.065 | 0.065 | 0.053 |
| 48 | 0.51 | 0 | 0.009 | 0 | 0 | 0.014 | 0.04 | 0.040 | 0.031 |
| | I9 | | | | | | | | |
| | Org | | | Dual | | | MC | | |
| Hap | Org | Dual | MC | Org | Dual | MC | Org | Dual | MC |
| 0 | 0.48 | 0.477 | 0.368 | 0.065 | 0.045 | 0.162 | 0 | 0.030 | 0.036 |
| 43 | 0.325 | 0.340 | 0.277 | 0.11 | 0.089 | 0.128 | 0 | 0 | 0.035 |
| 62 | 0.095 | 0.071 | 0.081 | 0.47 | 0.528 | 0.240 | 0.125 | 0.103 | 0.350 |
| 11 | 0.1 | 0.112 | 0.170 | 0.265 | 0.253 | 0.155 | 0.085 | 0.089 | 0.120 |
| 8 | 0 | 0 | 0.025 | 0.055 | 0.078 | 0.133 | 0.335 | 0.296 | 0.209 |
| 42 | 0 | 0 | 0.028 | 0.035 | 0 | 0.107 | 0.265 | 0.300 | 0.165 |
| 56 | 0 | 0 | 0.035 | 0 | 0 | 0.051 | 0.115 | 0.133 | 0.059 |
| 48 | 0 | 0 | 0.015 | 0 | 0.008 | 0.024 | 0.075 | 0.049 | 0.028 |

## PATENT COOPERATION TREATY

# PCT

DECLARATION OF NON-ESTABLISHMENT OF INTERNATIONAL SEARCH REPORT

(PCT Article 17(2)(a), Rules 13*ter*.1(c) and 39)

| Applicant's or agent's file reference<br>PCT04007 | **IMPORTANT DECLARATION** | Date of mailing *(day/month/year)*<br>30 November, 2004 (30.11.04) |
|---|---|---|
| International application No.<br>PCT/JP2004/013075 | International filing date *(day/month/year)*<br>08 September, 2004 (08.09.04) | (Earliest) Priority Date *(day/month/year)* |

International Patent Classification (IPC) or both national classification and IPC
Int.Cl⁷ G06F19/00

Applicant
Kabushiki Kaisha Jeneshisu Tekunorojizu

---

This International Searching Authority hereby declares, according to Article 17(2)(a), that **no international search report will be established** on the international application for the reasons indicated below.

1. ☒ The subject matter of the international application relates to:

    a. ☒ scientific theories.

    b. ☐ mathematical theories.

    c. ☐ plant varieties.

    d. ☐ animal varieties.

    e. ☐ essentially biological processes for the production of plants and animals, other than microbiological processes and the products of such processes.

    f. ☐ schemes, rules or methods of doing business.

    g. ☐ schemes, rules or methods of performing purely mental acts.

    h. ☐ schemes, rules or methods of playing games.

    i. ☐ methods for treatment of the human body by surgery or therapy.

    j. ☐ methods for treatment of the animal body by surgery or therapy.

    k. ☐ diagnostic methods practised on the human or animal body.

    l. ☐ mere presentations of information.

    m. ☐ computer programs for which this International Searching Authority is not equipped to search prior art.

2. ☐ The failure of the following parts of the international application to comply with prescribed requirements prevents a meaningful search from being carried out:

    ☐ the description      ☐ the claims      ☐ the drawings

3. ☐ The failure of the nucleotide and/or amino acid sequence listing to comply with the standard provided for in Annex C of the Administrative Instructions prevents a meaningful search from being carried out:

    ☐ the written form has not been furnished or does not comply with the standard.

    ☐ the computer readable form has not been furnished or does not comply with the standard.

4. ☐ The failure of the tables related to the nucleotide and/or amino acid sequence listing to comply with the technical requirements provided for in Annex C-*bis* of the Administrative Instructions prevents a meaningful search from being carried out:

    ☐ the written form has not been furnished.

    ☐ the computer readable form has not been furnished or does not comply with the technical requirements.

5. Further comments:

---

| Name and mailing address of the ISA/<br>Japanese Patent Office | Authorized officer |
|---|---|
| Facsimile No. | Telephone No. |

Form PCT/ISA/203 (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003288346 A